# EUROPEAN PATENT SPECIFICATION

(11) **EP 0 830 088 B1**
(45) Date of publication and mention of the grant of the patent: **22.01.2003**
(21) Application number: 96918208.8
(22) Date of filing: 05.06.1996
(51) Int. Cl.: A61B 18/00

(54) **SURGICAL CHANNEL FORMING DEVICE WITH PENETRATION LIMITER**
MIT EINDRINGTIEFEBEGRENZER VERSEHENE CHIRURGISCHE VORRICHTUNG ZUM ERZEUGEN VON KANÄLEN
DISPOSITIF CHIRURGICAL DE FORMATION DE CANAUX A LIMITEUR DE PENETRATION

(30) Priority: 07.06.1995 US 482125; 11.01.1996 US 584957
(43) Date of publication of application: 25.03.1998
(73) Proprietor: ECLIPSE SURGICAL TECHNOLOGIES, INC., Sunnyvale, California 94089 (US)
(72) Inventor: JAVIER, Manuel A., Jr., Santa Clara, CA 95050 (US); PEARCE, Stephen, B., Fremont, CA 94555 (US); KESTEN, Randy, J., Mountain View, CA 94043 (US); PAYNE, Sam, G., Santa Clara, CA 95051 (US); GERTNER, Kevin, Campbell, CA 95008 (US)
(74) Representative: Barz, Peter, Dr.
(86) International application number: US9609160
(87) International publication number: WO96039965

(56) References cited:
- EP-A- 0 292 621
- EP-A- 0 542 428
- EP-A- 0 622 051
- WO-A-94/20037
- DE-A- 3 443 073
- GB-A- 2 227 103
- US-A- 4 658 817
- US-A- 4 660 571
- US-A- 5 389 096

## Description

### BACKGROUND OF THE INVENTION

This invention is directed to the formation of one or more channels into the wall of a patient's heart which may be used to increase blood flow to heart tissue experiencing ischemic conditions, for the delivery of therapeutic or diagnostic agents to various locations in the patient's heart or for a variety of other utilities.

The formation of a channel in a patient's ventricular wall to increase the blood to flow to a patient's heart tissue is called trans myocardial revascularization. The first clinical trials of the trans myocardial revascularization process were performed by Mirhoseini *et al.* See for example the discussions in Lasers in General Surgery (Williams & Wilkins; 1989), pp 216-223. Other early disclosures of this procedure is found in an article by Okada *et al.* in Kobe J. Med. Sci 32, 151-161, October 1986 and U.S. Patent 4,658,817 (Hardy). These early references describe intraoperative revascularization procedures which require an opening in the chest wall and include formation of the channels through the epicardium.

U.S. Patent No. 5,554,152 (Aita *et al*.) describes a system for trans myocardial revascularization which is introduced through the chest wall. In U.S. Patent No. 5,389,096 (Aita *et al.*) a percutaneous method is described for forming a channel in a patient's ventricular wall wherein an optical fiber device is advanced through a peripheral artery such as the femoral artery, through the aorta into the patient's left ventricle. Within the left ventricle, the distal end of the optical fiber device is directed toward a desired location on the patient's endocardium and urged against the endocardial surface while a laser beam is emitted from its distal end to form the channel. The depth of penetration of the distal end of the laser device is affected by the force applied by the distal end to the tissue into which the channel is being formed. Because of the nature of the environment, i.e. fluid currents, the moving heart surface and the uneven surface of the patient's endocardium, controlling the force applied to the endocardial tissue by the end of the laser device can be quite difficult. Complete penetration through the ventricular wall from within the ventricular chamber is not desirable.

The present invention minimizes the difficulties with these prior channel forming devices.

### SUMMARY OF THE INVENTION

The present invention is directed to an improved device for forming a channel in a ventricular wall of a patient's heart and particularly in the free-wall defining in part the left ventricle of the patient's heart.

The channel forming device of the invention includes an elongated shaft with a proximal and distal shaft sections. A means to form a channel in the ventricular wall of the is provided on the distal shaft section and a means to limit the depth of penetration of the distal shaft section into heart tissue during the formation of the channel therein.

The invention includes an elongated optical fiber having a proximal end and a distal end and an optical probe member disposed about and secured to the distal extremity of the optical fiber. The device is provided with at least one radially expandable arm spaced from the distal end of the device which acts as a stop to limit the penetration or forward motion of the operative end of the device into the ventricular wall during channel formation, which in turns controls the depth of the channel into the ventricular wall. The radially expandable arms fold back when the channel forming device is advanced within a guiding or delivery catheter to the ventricular chamber, but expand outwardly when the distal section exits the distal end of the guiding or delivery catheter within the chamber. The stopping surface is spaced from the distal end of the probe member the desired penetration distance for the distal end of the device. By providing the means to control the depth of penetration, there is no need for concern about the pressure applied by the physician to the channel forming device affecting the depth of penetration which may lead to the complete penetration of the ventricular wall.

The optical probe member preferably has an interior chamber into which the distal extremity of the optical fiber extends and a tubular support member is secured to the proximal portion of the probe member and a distal portion of the optical fiber extending out the proximal end of the probed member to ensure the integrity of the probe member and optical fiber during the channel forming procedure. The tubular support member may be shrunk fit onto the probe member or it may be bonded by a suitable adhesive.

In a presently preferred device for forming channels in the ventricular wall from within the ventricular chamber which is percutaneously introduced into the patient's vascular system, the probe member length is about 3 to about 20 mm and the length of the portion of the probe member which extends out the distal end of the tubular support member is about 1 to about 5 mm. Generally, at least about 1 mm of the proximal portion of the probe member, preferably at least about 2 mm thereof, is secured by the tubular support member to ensure holding the probe member in the case of a fractured probe member. The proximal portion of the tubular support member secured to the distal end of the optical fiber should be at least about the same length as described above for the distal portion, although generally it will be longer. For an interoperative device which is designed to form channels from the exterior of the patient's heart the dimensions may be significantly larger than those set forth above for a percutaneous device.

An adapter is provided on the proximal end of the device which is configured to connect the proximal end of the optical fiber in an optical transmission relationship with a laser source.

While forming a passageway through the wall of the patient's heart for the purpose of revascularization is of significant importance, the passageway formed into the heart wall may be used for other purposes. For example, therapeutic or diagnostic agents may be introduced into the channel for delivery to the patient's endocardium or myocardium. The therapeutic or diagnostic agent may be incorporated into a biocompatible matrix deposited within the channel for delivery or release over an extended period.

These and other advantages of the invention will become more apparent from the following detailed description of the invention, when taken in conjunction with the accompanying exemplary drawings.

### BRIEF DESCRIPTION OF THE DRAWINGS

Fig. 1 is an elevational view, partially in section, of a channel forming device embodying features of the present invention.
Fig. 2 is a transverse cross-sectional view of the channel forming device shown in Fig. 1, taken along the lines 2-2.
Fig. 3 is a transverse cross-sectional view of the channel forming device shown in Fig. 1, taken along the lines 3-3.
Fig. 4 is a transverse cross-sectional view of the channel forming device shown in Fig. 1, taken along the lines 4-4.
Fig. 5 is an end view of the device shown in Fig. 1.
Fig. 6 is an elevational view of the device shown in Fig. 1 disposed within a guiding catheter.
Fig. 7 is an elevational view, partially in section, of the device shown in Fig. 1 positioned adjacent to a patient's heart wall with the optical probe member disposed within the formed channel.
Fig. 8 is an elevational view, partially in section, of an alternative channel forming device not being an embodiment of the present invention.
Fig. 9 is a transverse cross-sectional view of the channel forming device shown in Fig. 8, taken along the lines 9-9.
Fig. 10 is a transverse cross-sectional view of the channel forming device shown in Fig. 8, taken along the lines 10-10.
Fig. 11 is a transverse cross-sectional view of the channel forming device shown in Fig. 8, taken along the lines 11-11.

### DETAILED DESCRIPTION OF THE INVENTION

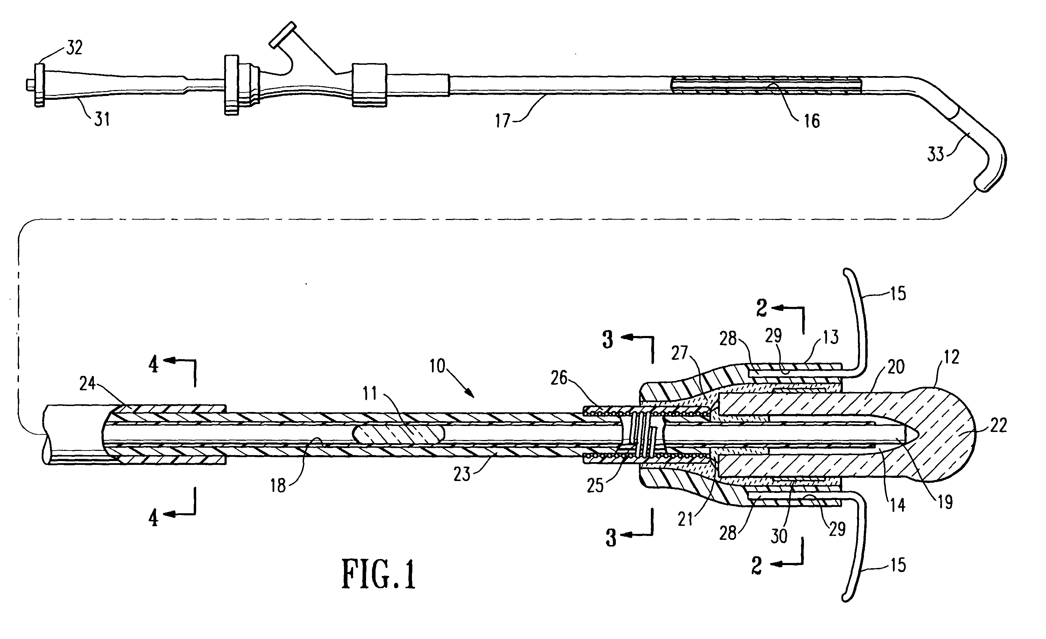

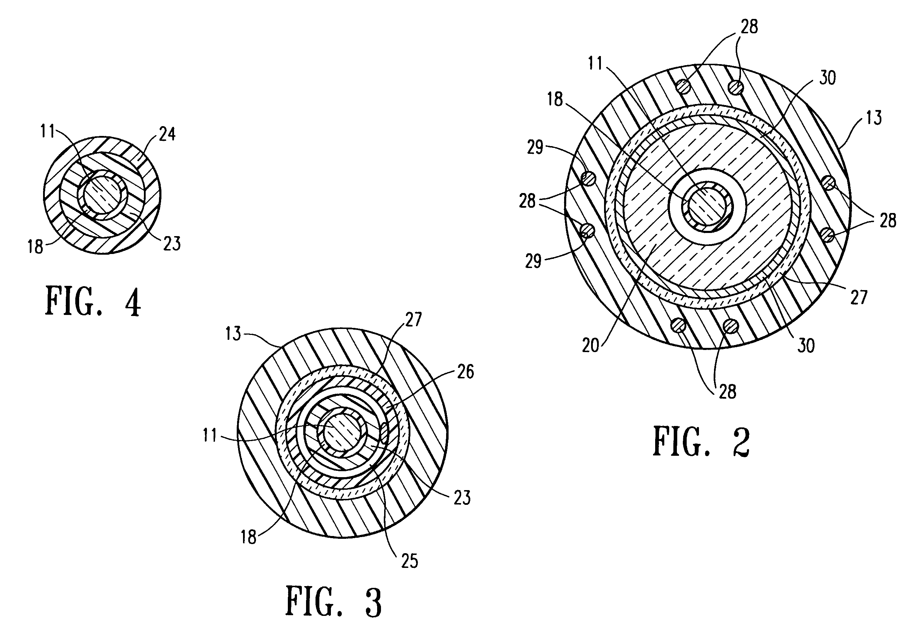

In Figs. 1 - 4 a channel forming device 10 is shown which embodies features of the invention. The device 10 includes an elongated optical fiber 11, an elongated probe tip 12 disposed about and secured to the distal extremity of the optical fiber, and an outer tubular support member or sleeve 13 secured to the exterior of the proximal extremity of the probe 12 and a distal portion of the optical fiber which is not disposed in the interior chamber 14 of the probe 12. A plurality of radially extending arms 15 are provided proximal to the distal end of the probe tip to limit the penetration of the probe tip into the patient's ventricular wall. The channel forming device 10 is slidably disposed within the inner lumen 16 of guiding catheter 17. A positioning catheter (not shown) may be disposed between channel forming device 10 and the guiding catheter 17 which can facilitate closer placement of the probe tip 12 onto the endocardium of the ventricular wall.

The exterior of the optical fiber 11 is provided with a fluoropolymeric cladding 18 along its length except for a distal portion 19 which extends into the distal end of the interior chamber 14 of the probe tip 12.

The elongated probe tip 12 has a cylindrical body 20 which is bonded to the distal end of the optical fiber 11 by adhesive 21. The probe tip 12 has a distal end 22 which acts as a lens to control laser energy emitted from the distal end of the optical fiber to a location immediately distal to the lens to ensure formation a channel of a desired size in the ventricular wall. A fluoropolymer buffer 23 is disposed about the optical fiber 11 proximal to the body of adhesive 21 and extends proximally along essentially the remainder of the optical fiber. An outer jacket 24 is disposed about the fluoropolymer buffer 23 along most of its length, and terminates about 10 cm from the proximal end of the outer tubular support member 13. A helical coil 25 formed of high strength material such as stainless steel, NITINOL and the like is disposed between the fluoropolymer buffer 23 and the proximal end of the outer tubular support member 13 and has a jacket 26 formed of suitable plastic material such al polyethylene terephthalate (PET). The coil 25 and jacket 26 together facilitate an effective bond between the fluoropolymer buffer 23 and the outer tubular support member 13. Adhesive 27 bonds the outer tubular support member 13 to the coil jacket 26.

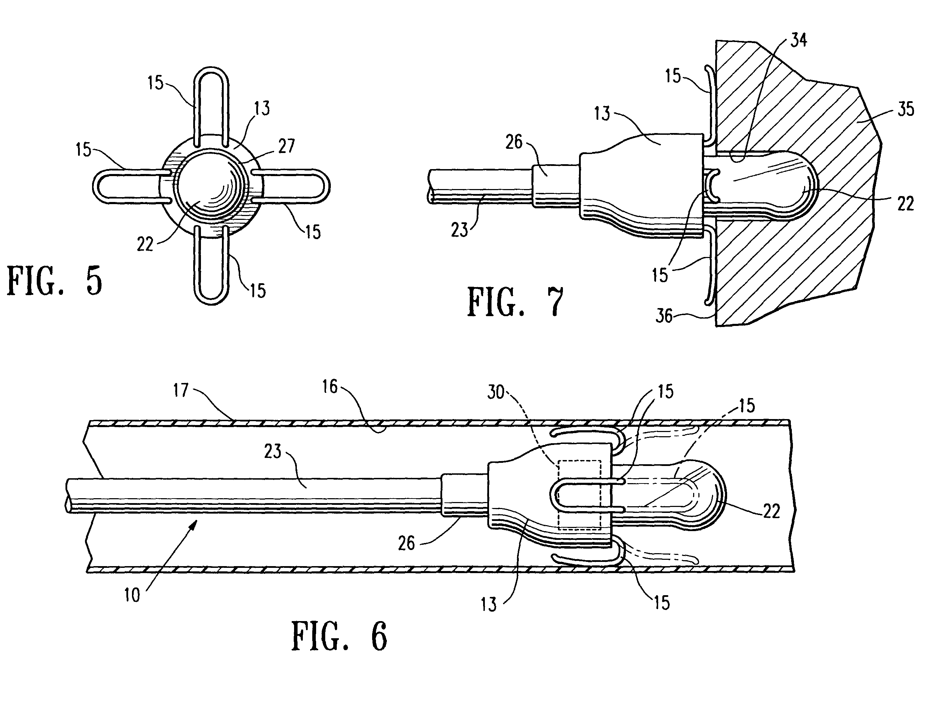

The radially extending arms 15 are U-shaped flexible wire members with the free ends 28 embedded within the outer tubular support member 13. Preferably, the outer tubular support member 13 is provided with lumens 29 which receive the free ends 26 of the arms 15 and are secured therein by a suitable adhesive (not shown). Other means may be utilized to secure the free ends 28 of the arms 15 between the probe tip 12 and the exterior of the outer tubular support member 13. When expanded radially, the arms 15 act to limit the penetration of the probe tip 12 into the channel as it is being formed in the ventricular wall and thus controls the depth of the channel formed. As is shown in Fig. 6, the arms 15 are folded backwardly while the probe tip 12 of optical fiber device 10 is disposed within the inner lumen 16 of the guiding catheter 17. Alternatively, they may be folded forwardly as shown in phantom in Fig. 6. Preferably, the arms 15 are formed of a NiTi alloy having superelastic characteristics at body temperature to facilitate the folding thereof within the guiding catheter 17 and the radial expansion thereof once the probe tip 12 extends out of the distal end of the guiding catheter. Folding the arms 15 causes the stress induced transformation of the austenite phase of the NiTi alloy in the bent portions to the martensite phase and release of the arms to allow for their radial expansion causes the transformation of the martensite phase back to the austenite phase.

A radiopaque band 30 may be secured to the exterior of the cylindrical body 20 by the adhesive 27 to facilitate the fluoroscopic observation of the probe tip 12 during the procedure. The band may be formed of a wide variety of metallic components including gold, platinum iridium and the like.

The proximal end of the device 10 is provided with a connector 31 which has a rotatable, internally threaded collar 32 which facilitates an optical connection of the proximal end of the optical fiber 11 with a source of laser energy.

The distal end 33 of the guiding catheter 17 is preferably formed into a desired shape which directs the distal extremity of the optical fiber 11 and the probe tip 12 onto a desired location on the surface of the free ventricular wall of the patient's heart. If a positioning catheter is employed, its distal end may likewise be formed with a desirable shape to facilitate directing the probe tip to the desired location.

The various components of the device 10 may be formed of a wide variety of conventional materials used in the construction of intravascular catheters and other intracorporeal devices. The contemplated materials of construction and the sources thereof for one presently preferred embodiment are provided in the following table.

| COMPONENT | MATERIAL | SUPPLIER |
|---|---|---|
| Proximal Optical Connector | Various | Amphenol Corporation Lisle, IL and Spectran¹ Specialty Optics, Co. Avon, CT |
| Jacket (24) | Pebax 7233 tubing with 3% TiO₂ | North American Infinity Extrusions and Engineering, Inc. Santa Clara, CA 95054 |
| Tubular Support Member (13) | Nylon 12, 1/8" | Guidant Corporation 3200 Lakeside Dr. Santa Clara, CA 95052 |
| UV-Cured Adhesive (20) | Urethane Oligomer (197-M) Acrylate | Dymax Corp. Torrington, CT |
| PET Shrink Tubing (26) | Polyethylene Terephthalate | Advanced Polymers, Inc. Salem, NH |
| Probe (12) | Fused Quartz | Polymicro Technologies, Inc. Phoenix, AZ |
| Optical Fiber Buffer (23) | Tefzel® | Spectran¹ Specialty Optic Co. Avon, CT |
| Optical Fiber Cladding (18) | Proprietary Flouropolymer Acrylate | Spectran¹ Specialty Optic Co. Avon, CT |
| Optical Fiber (11) | Fused Silica (Low OH⁻) | Spectran¹ Specialty Optic Co. Avon, CT |

The overall length of a channel forming device in accordance with the present invention is about 200 to about 400 cm with a typical value being about 350 cm. The actual length being determined by the location of the source of laser energy. The operative distal portion of the device, i.e. the portion which is inserted into the patient is about 10 to about 60 cm in length. The probe tip for percutaneous use is about 3 to about 10 mm in length with the length of the exposed distal portion which extends out of the tubular support member being about 1 to about 5 mm, preferably about 2 to about 4 mm. For intraoperative use the probe tip should be about 5 to about 50 mm in length with about 2 to about 30 mm, preferably about 10 to about 25 mm, extending out of the tubular support member. The outer diameter of the probe tip is about 1 to about 3 mm, preferably about 1.5 to about 2 mm, and is measured at the widest portion of the bulbous tip which forms the lens. The outer diameter of the coating or jacket on the probe tip is essentially the same as the bulbous tip. The length of the outer tubular support member is about 0.3 to about 40 cm, preferably about 0.5 to about 30 cm and the length of the radial extension of the arms 15 is about 0.5 to about 2 mm.

Fig. 7 illustrates the use of the channel forming device wherein the probe tip 12 is disposed within the channel 34 in the ventricular wall 35. The arms 15 rest upon the ventricular surface 36 limiting the penetration of the probe tip 12.

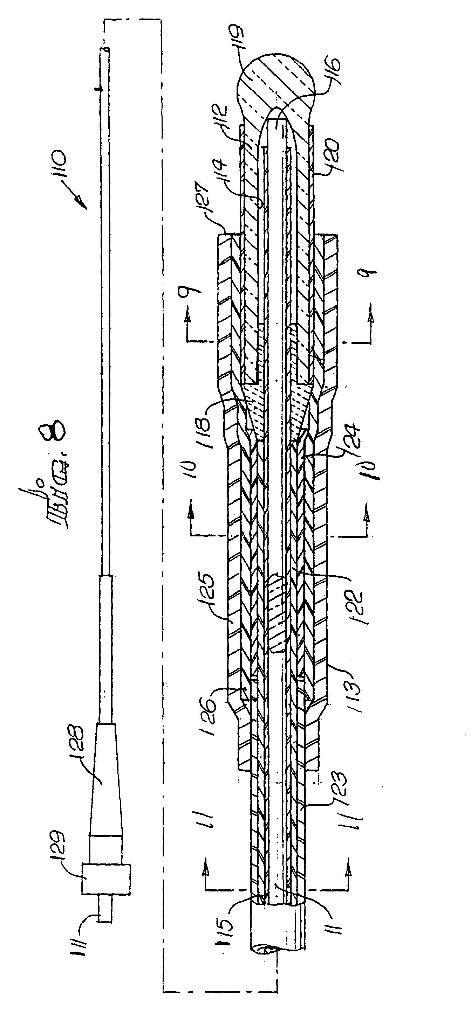

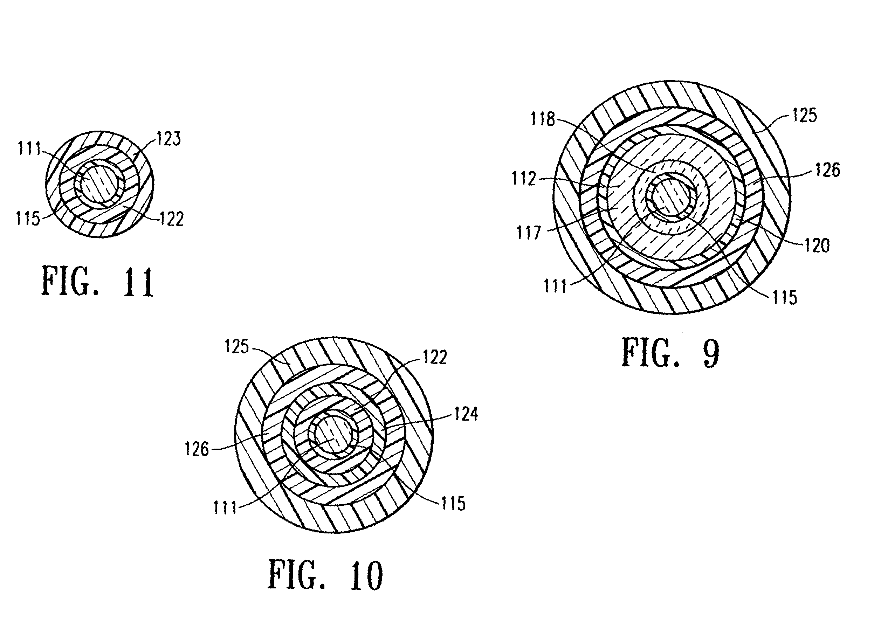

Figs. 8 - 11 show a channel forming device 110 which is not an embodiment of the invention. The device 110 includes an elongated optical fiber 111, an elongated probe 112 disposed about and secured to the distal extremity of the optical fiber, and an outer tubular support member 113 secured to the exterior of the proximal extremity of the probe 112 and a distal portion of the optical fiber which is not disposed in the interior chamber 114 of the probe 112.

The exterior of the optical fiber 111 is provided with a fluoropolymeric cladding 115 along its length except for the distal portion 116 which extends into the distal portion of the interior chamber 114. The elongated probe 112 has a cylindrical body 117 which is bonded to the optical fiber 111 by adhesive 118. The probe 112 has a bulbous distal end 119 which acts as a lens to control laser energy emitted from the distal end of the optical fiber to a location immediately distal to the lens to ensure formation a channel of a desired size. The cylindrical body 117 is provided with a coating or jacket 120 of suitable plastic material which will aid in the bonding of the outer tubular support member 113, strengthen the probe 112 and maintain the integrity of the probe, if the lens material fractures. The plastic material can be a heat shrinkable materials such as polyethylene terephthalate (PET) or polyethylene. The optical fiber 111 within the elongated probe 112 is provided with a body of adhesive 118 which prevents relative longitudinal movement between the optical fiber and the elongated probe 112. A fluoropolymer buffer 122 is disposed about the optical fiber 111 proximal to the body of adhesive 118 and extends proximally along essentially the remainder of the optical fiber. An outer jacket 123 is disposed about the fluoropolymer buffer 122 along its length, and terminates within the outer support tubular support member 113 proximal to the elongated probe 112. Filler tubing 124 is provided on the exterior of the buffer 122 and generally extends from the distal end of jacket 123 to the adhesive 118.

The outer tubular support member 113 has an outer and inner tubular elements 125 and 126 with the distal ends thereof forming a annular shoulder 127 which acts to limit the penetration of the probe 112 into the channel as it is being formed and thus the depth of the channel. The outer tubular element 125 is longer than the inner tubular element 126 and the proximal end of the outer tubular member is secured to the exterior of jacket 123. The inner tubular member 126 is secured to the filler shrink tubing 124 and the coating 120 on the cylindrical body 117 of the elongated probe 112. The inner and outer tubular elements 125 and 126 can be formed of heat shrinkable materials such as polyethylene so that these elements can be heat shrunk onto the proximal extremity of the probe 111 and the distal extremity of the optical fiber which does not extend into the probe 112 and secure these members together. Other means of securing the outer tubular support member 113 to the optical fiber 111 and the elongated probe 112 may be employed, such as a suitable adhesive or insert injection molding.

The proximal end of the device 110 is provided with a connector 128 which has a rotatable, internally threaded collar 129 which facilitates an optical connection with a source of laser energy.

While the present invention has been described herein primarily in terms of a laser based channel forming device which is percutaneously introduced into the patient's vascular system and then advanced therein until the operative end is disposed within a chamber of the patient's heart, those skilled in the art will recognize that the device of the invention may be utilized in an interoperative procedure where the device is introduced into the patient's chest cavity and the channel is formed through the patient's epicardium. In this instance, however, the dimensions of the device may have to be changed to accommodate the slightly different delivery system.

## Claims

1. A device (10) for forming a channel into a ventricular wall of a patient's heart comprising:
a) an elongated shaft having a proximal shaft section and a distal shaft section,
b) an elongated optical fiber (11) having proximal and distal ends and
c) an optical probe member (12) disposed about and secured to the distal extremity of the optical fiber (11), said optical probe member having a distal end (22) which acts as a lens to control laser energy emitted from the distal end of the optical fiber to a location immediately distal to the lens to ensure formation of a channel of a desired size in the ventricular wall, **characterized by**
d) at least one radially expandable arm (15) proximal to the distal end (22) of the optical probe member (12) to limit the depth of penetration of said optical probe member into the ventricular wall during the formation of the channel therein.

2. The device of claim 1 wherein the radially expandable arm (15) is foldable over the exterior of the distal shaft section to facilitate advancement of the device through an inner lumen (16) of a delivery catheter (17).

3. The device of claim 1 or 2 wherein said optical probe member (12) has an interior chamber (14) into which the distal extremity of the optical fiber (11) extends with the distal end (19) of the optical fiber being in an optical transmitting relationship with the lens on the distal end (22) of the optical probe member (12).

4. The device of claim 3 wherein a tubular support member (13) having distal and proximal extremities is provided, the distal extremity being disposed about and secured to a proximal portion of the optical probe member (12) and the proximal extremity being disposed about and secured to a distal portion of the optical fiber (11) which is not disposed within the interior chamber (14) of the optical probe member (12).

5. The device of claim any one of claims 1-4 wherein the optical fiber (11) is provided with a fluoropolymer cladding (18) over essentially its entire length excluding its distal tip which is essentially free of such cladding.

6. The device of claim 5 wherein a helical coil (25) is disposed between the fluoropolymer cladding (18) on the optical fiber (11) and the proximal end of the tubular support member (13).

7. The device of claim 6 wherein the helical coil (25) is provided with a polymer jacket (26) to facilitate securing the outer tubular member (13) to the optical fiber (11).

8. The device of any one of claims 1-7 wherein the optical probe member (12) is 3 to 10 mm in length for percutaneous use.

9. The device of any one of claims 1-7 wherein the optical probe member (12) is 5 to 50 mm in length for intraoperative use.

10. The device of any one of claims 4-7 wherein the length of the optical probe member (12) extending out of the tubular support member (13) is 1 to 30 mm, preferably 2 to 25 mm.

## Patentansprüche

1. Vorrichtung (10) zum Erzeugen eines Kanals in einer Ventrikelwand des Herzens von einem Patienten, umfassend:
a) einen länglichen Schaft mit einem proximalen Schaftabschnitt und einem distalen Schaftabschnitt,
b) eine längliche optische Faser (11) mit proximalen und distalen Enden und
c) ein optisches Sondenelement (12), das um das distale Ende der optischen Faser (11) herum angeordnet und daran befestigt ist, wobei das optische Sondenelement ein distales Ende (22) aufweist, das als Linse wirkt, um Laserenergie, die vom distalen Ende der optischen Faser abgestrahlt wird, auf eine Stelle unmittelbar distal zur Linse zu regulieren, um die Erzeugung eines Kanals von einer gewünschten Größe in der Ventrikelwand sicherzustellen, **gekennzeichnet durch**
d) mindestens einen radial ausdehnbaren Arm (15) proximal zum distalen Ende (22) des optischen Sondenelements (12), um die Eindringtiefe des optischen Sondenelements in die Ventrikelwand während der Erzeugung des Kanals zu begrenzen.

2. Vorrichtung nach Anspruch 1, worin der radial ausdehnbare Arm (15) über die Außenseite des distalen Schaftabschnitts umklappbar ist, um das Verschieben der Vorrichtung durch ein Innenlumen (16) eines Zuführungskatheters (17) zu erleichtern.

3. Vorrichtung nach Anspruch 1 oder 2, worin das optische Sondenelement (12) eine Innenkammer (14) aufweist, in die sich das distale Ende der optischen Faser (11) erstreckt, wobei das distale Ende (19) der optischen Faser in einer optischen Übertragungsbeziehung zu der Linse am distalen Ende (22) des optischen Sondenelements (12) steht.

4. Vorrichtung nach Anspruch 3, worin ein röhrenförmiges Trägerelement (13) mit distalen und proximalen Enden vorgesehen ist, wobei das distale Ende um einen proximalen Bereich des optischen Sondenelements (12) herum angeordnet und daran befestigt ist und das proximale Ende um einen distalen Bereich der optischen Faser (11) herum angeordnet und daran befestigt ist, der sich nicht in der Innenkammer (14) des optischen Sondenelements (12) befindet.

5. Vorrichtung nach irgendeinem der Ansprüche 1 bis 4, worin die optische Faser (11) im wesentlichen über ihre gesamte Länge mit einer Fluorpolymer-Umhüllung (18) versehen ist, ihre distale Spitze ausgenommen, die im wesentlichen frei von einer derartigen Umhüllung ist.

6. Vorrichtung nach Anspruch 5, worin eine Spiralwendel (25) zwischen der Fluorpolymer-Umhüllung (18) auf der optischen Faser (11) und dem proximalen Ende des röhrenförmigen Trägerelements (13) angeordnet ist.

7. Vorrichtung nach Anspruch 6, worin die Spiralwendel (25) mit einer Polymerverkleidung (26) versehen ist, um die Befestigung des äußeren röhrenförmigen Elements (13) an die optische Faser (11) zu erleichtern

8. Vorrichtung nach irgendeinem der Ansprüche 1 bis 7, worin das optische Sondenelement (12) eine Länge von 3 bis 10 mm für die perkutane Anwendung aufweist.

9. Vorrichtung nach irgendeinem der Ansprüche 1 bis 7, worin das optische Sondenelement (12) eine Länge von 5 bis 50 mm für die intraoperative Anwendung aufweist.

10. Vorrichtung nach irgendeinem der Ansprüche 4 bis 7, worin die Länge des optischen Sondenelements (12), die aus dem röhrenförmigen Trägerelement (13) ragt, 1 bis 30 mm, vorzugsweise 2 bis 25 mm, beträgt.

## Revendications

1. Dispositif (10) permettant de former un canal dans la paroi ventriculaire du coeur d'un patient, comportant :
a) une tige allongée possédant une partie proximale de tige et une partie distale de tige,
b) une fibre optique allongée (11) possédant une extrémité distale et une extrémité proximale,
c) et une sonde optique (12), disposée autour et assujettie à l'extrémité distale de la fibre optique (11), cette sonde optique ayant une extrémité distale (22) qui joue le rôle d'une lentille permettant de réguler l'énergie laser émise depuis l'extrémité distale de la fibre optique vers un endroit immédiatement distal par rapport à la lentille de manière à assurer la formation d'un canal de taille voulue dans la paroi ventriculaire,
et **caractérisé par**
d) au moins une branche radialement déployable (15), en position proximale par rapport à l'extrémité distale (22) de la sonde optique (12), et servant à limiter la profondeur de pénétration de ladite sonde optique dans la paroi ventriculaire au cours de la formation d'un canal dans celle-ci.

2. Dispositif conforme à la revendication 1, dans lequel la branche radialement déployable (15) peut se replier sur l'extérieur de la partie distale de la tige, pour faciliter l'avance du dispositif dans la lumière interne (16) d'un cathéter d'amenée (17).

3. Dispositif conforme à la revendication 1 ou 2, dans lequel ladite sonde optique (12) comporte une chambre intérieure (14) dans laquelle s'étend l'extrémité distale de la fibre optique (11), laquelle fibre optique se trouve, par son extrémité distale (19), en relation de transmission optique avec la lentille formée par l'extrémité distale (22) de la sonde optique (12).

4. Dispositif conforme à la revendication 3, dans lequel il y a un élément tubulaire de support (13) possédant une extrémité distale et une extrémité proximale, l'extrémité distale étant disposée autour et assujettie à une partie proximale de la sonde optique (12), et l'extrémité proximale étant disposée autour et assujettie à une partie distale de la fibre optique (11) qui ne se trouve pas dans la chambre intérieure (14) de la sonde optique (12).

5. Dispositif conforme à l'une des revendications 1 à 4, dans lequel la fibre optique (11) est pourvue d'un revêtement (18) en polymère fluoré qui s'étend pratiquement sur toute la longueur de la fibre, à l'exclusion de la pointe distale de celle-ci, qui est pratiquement dépourvue d'un tel revêtement.

6. Dispositif conforme à la revendication 5, dans lequel un enroulement hélicoïdal (25) est disposé entre le revêtement (18) en polymère fluoré de la fibre optique (11) et l'extrémité proximale de l'élément tubulaire de support (13).

7. Dispositif conforme à la revendication 6, dans lequel l'enroulement hélicoïdal (25) est muni d'une enveloppe (26) en polymère qui sert à faciliter l'assujettissement de l'élément tubulaire extérieur (13) à la fibre optique (11).

8. Dispositif conforme à l'une des revendications 1 à 7, dans lequel 1a sonde optique (12) est longue de 3 à 10 mm et destinée à être employée en mode percutané.

9. Dispositif conforme à l'une des revendications 1 à 7, dans lequel la sonde optique (12) est longue de 5 à 50 mm et destinée à être employée en mode opératoire interne.

10. Dispositif conforme à l'une des revendications 4 à 7, dans lequel la partie de la sonde optique (12) qui dépasse hors de l'élément tubulaire de support (13 est longue de 1 à 30 mm, et de préférence, de 2 à 25 mm.
